# EUROPÄISCHE PATENTSCHRIFT

(11) **EP 0 672 647 B1**
(45) Veröffentlichungstag und Bekanntmachung des Hinweises auf die Patenterteilung: **09.12.1998**
(21) Anmeldenummer: 95103317.4
(22) Anmeldetag: 08.03.1995
(51) Int. Cl.: C07C 227/08

(54) **Verfahren zur Herstellung von Aminoethylglycin**
Process for the preparation of aminoethylglycine
Procédé pour la préparation d'aminoéthylglycine

(30) Priorität: 14.03.1994 DE 4408530
(43) Veröffentlichungstag der Anmeldung: 20.09.1995
(73) Patentinhaber: HOECHST AKTIENGESELLSCHAFT, 65929 Frankfurt am Main (DE)
(72) Erfinder: Breipohl, Gerhard, Dr., D-60529 Frankfurt (DE)

(56) Entgegenhaltungen:
- EP-A- 0 187 130
- JOURNAL OF MEDICINAL CHEMISTRY, Bd. 33, 1990 WASHINGTON US, Seiten 2590-2595, W.S. SAARI ET AL. 'Cyclization-Activated Prodrugs. Basic Esters of 5-Bromo-2'-deoxyuridine'
- INT. J. PEPTIDE PROTEIN RES. , Bd. 23, 1984 Seiten 203-211, E.P. HEIMER ET AL. 'Synthesis of analogs and oligomers of N-(2-aminoethyl)glycine and their gastrointestinal absorption in the rat'

## Beschreibung

Aminoethylglycin ist eine ungewöhnliche Aminosäure, die unter anderem als zentraler Baustein bei der Herstellung sogenannter PNA's eine wichtige Rolle spielt. Die bekannten Verfahren der Herstellung bedienen sich der Alkylierung von Diaminoethan durch Halogenessigsäurederivate (E.P. Heimer et al. , Int. J. Peptide Protein Res. 23, 1984, 203 - 211) oder der Reaktion von Diaminoethan mit Formaldehyd und Natriumcyanid (US-A- 2 387 735). Bei diesen genannten Verfahren entstehen jedoch Salze sowie unerwünschte Nebenprodukte; außerdem ist die Aufarbeitung mühsam. Außerdem beschreibt J.M. Tien et al. (J. Am. Chem. Soc. 77, 1955, 6996 - 6998) die Synthese von N-Alkylglycinen durch Reaktion von Monoamin mit Glyoxylsäureester und anschließender Verseifung.

Saari et al. (J. Med. Chem., 1990, 33, 2590-2595) beschreiben basische Ester von 5-Brom-2'-deoxyuridin und deren Synthese. N-Ethylaminoethylglycin wird dabei als Zwischenprodukt durch reduktive Aminierung von Glyoxylsäure mit N-Ethylethylendiamin in Ethanol in Gegenwart eines Pd/C-Katalysators erhalten.

Überraschenderweise haben wir nun gefunden, daß sich Aminoethylglycin in einem sehr einfachen Verfahren aus Diaminoethan und Glyoxylsäure durch reduktive Aminierung herstellen läßt. Das Verfahren zeichnet sich durch eine einfache Durchführung, Vermeidung von salzhaltigen Nebenprodukten und eine leichte Reinigung des Rohproduktes aus.

Das Verfahren zur Herstellung von Aminoethylglycin ist dadurch gekennzeichnet, daß man Diaminoethan mit Glyoxylsäure unter Kühlung und Rühren im Verhältnis von 2-10 : 1, bevorzugt 3-5 : 1, in Wasser, niederem Alkohol (wie Methanol, Ethanol, Isopropanol, bevorzugt Methanol) oder Wasser/Alkohol-Gemischen, die ca. 30 - 60 % vorstehend beschriebenen Alkohol enthalten, vermischt und mit Wasserstoff in Gegenwart eines Katalysators als Reduktionsmittel, wie beispielsweise Palladium auf Kohle, bei Normaldruck oder leicht erhöhtem Druck (bis 0,5 bar), bevorzugt bei 0,3 bar hydriert. Anschließend wird das Lösungsmittel und das überschüssige Diaminoethan, das gegebenenfalls erneut in die Reaktion eingesetzt werden kann, abdestilliert. Der Rückstand wird mehrmals mit Toluol ko-evaporiert und dann mit niederem Alkyl-Alkohol, wie Methanol oder Isopropanol, bevorzugt Methanol, beispielsweise bei Raumtemperatur bis 60 °C, bevorzugt bei Raumtemperatur bis 45 °C, behandelt, wobei Aminoethylglycin auskristallisiert. Unter Umständen kann noch etwas Kristallwasser vorhanden sein. Das Produkt fällt in guter Reinheit an, so daß die weitere Umsetzung, beispielsweise zum Ester, insbesondere Methylester, direkt mit dem Rohprodukt erfolgen kann, wobei eine deutlich geringere Alkoholmenge als in der Literatur, z.B. bei E.P. Heimer et al., Int. J. Peptide Protein Res. 23, 1984, 203 - 211, beschrieben bei der Veresterung eingesetzt werden kann.

Im folgenden sind einige Ausführungsarten des Verfahrens beschrieben, die die Vorteile des Verfahrens gegenüber den bisher beschriebenen hervorheben ohne jedoch darauf beschränkt zu sein.
Aminoethylglycin : (C₄H₁₀N₂O₂ 118.14)

### Beispiel 1:

4.6 g Glyoxylsäure-Monohydrat werden in 200 ml Wasser gelöst und dann unter Kühlung und Rühren 10.1 ml Diaminoethan dazugegeben. Die Mischung wird mit 2 g Katalysator( 10% Pd/C) versetzt und in der Schüttelente bei Normaldruck und Raumtemperatur hydriert. Nach Aufnahme von 1.3 l Wasserstoff ist die Reaktion beendet. Zur Aufarbeitung wird vom Katalysator abgesaugt und das Filtrat im Vakuum zur Trockene eingeengt. Der Rückstand wird zweimal mit wenig Toluol nachdestilliert und das ölige Rohprodukt mit 100 ml Isopropanol über Nacht stehengelassen wobei das Produkt auskristallisiert. Man rührt den Kristallbrei noch einmal gut durch und saugt dann ab und wäscht mit wenig Isopropanol nach.
Ausbeute: 4.18 g
R_{F}(n-Butanol/Essigsäure/Wasser/Ethylacetat = 1:1:1:1): 0.2
MS(DCI): 119 [M + H]⁺
NMR(D₂O): 2.92-3.18 ppm(m, 4H, CH₂-CH₂); 3.35 ppm(s, 2H, CH₂-CO)

### Beispiel 2:

334 ml Diaminoethan werden in 500 ml Ethanol gelöst und dann unter Kühlung und Rühren eine Lösung von 55.04 g Glyoxylsäure-Monohydrat in 100 ml Ethanol langsam zugegeben. Dann wird die Mischung mit 10 g Palladium auf Kohle versetzt und in der Schüttelente bei Normaldruck und Raumtemperatur hydriert. Nach Aufnahme von 10.9 l Wasserstoff ist die Reaktion beendet und der Katalysator wird abfiltriert. Das Filtrat wird im Vakuum eingeengt, zweimal mit Toluol nachdestilliert und der Rückstand mit 1 l Isopropanol versetzt und bei ca. 45°C ausgerührt. Der Niederschlag wird abgesaugt, mit wenig Isopropanol nachgewaschen und im Vakuum getrocknet.
Ausbeute: 47 g
R_{F}(n-Butanol/Essigsäure/Wasser/Ethylacetat = 1:1:1:1): 0.2
MS(DCI): 119 [M+H]⁺
NMR(D₂O): 2.92-3.18 ppm(m, 4H, CH₂-CH₂); 3.35 ppm(s, 2H, CH₂-CO)

### Beispiel 3:

In eine Mischung aus 500 ml Isopropanol und 500 ml Wasser werden unter Rühren und Kühlen nacheinander 66.4 ml Ethylendiamin und 55.1 ml einer wäßrigen 50%-igen Lösung von Glyoxylsäure gegeben. Dann wird die Mischung mit 2 g Palladium auf Kohle versetzt und in der Schüttelente bei Normaldruck und Raumtemperatur hydriert. Nach Aufnahme von 10.7 l Wasserstoff ist die Reaktion beendet und der Katalysator wird abfiltriert. Das Filtrat wird im Vakuum eingeengt, zweimal mit Toluol nachdestilliert und der Rückstand mit 250 ml Isopropanol versetzt und bei ca. 45°C ausgerührt. Der Niederschlag wird abgesaugt, mit wenig Isopropanol nachgewaschen und im Vakuum getrocknet.
Ausbeute: 48.7 g
R_{F}(n-Butanol/Essigsäure/Wasser/Ethylacetat = 1:1:1:1): 0.2
MS(DCl): 119 [M + H]⁺
NMR(D₂O): 2.92-3.18 ppm(m, 4H, CH₂-CH₂); 3.35 ppm(s, 2H, CH₂-CO)

### Beispiel 4:

161.1 g Glyoxylsäure-Monohydrat werden in 500 ml Wasser gelöst, dann 500 ml Methanol zugegeben und anschließend unter Kühlung und Rühren 348.5 ml Diaminoethan dazugegeben. Die Mischung wird mit 15 g Katalysator(5% Pd/C) versetzt und in der Schüttelente bei Normaldruck und Raumtemperatur hydriert. Nach Aufnahme von 37 l Wasserstoff ist die Reaktion beendet. Zur Aufarbeitung wird vom Katalysator abgesaugt und das Filtrat im Vakuum zur Trockene eingeengt. Der Rückstand wird dreimal mit Toluol (ca. 200 ml) nachdestilliert und das ölige Rohprodukt in 300 ml Methanol unter Erhitzen gelöst, abkühlen lassen und unter Zugabe von einigen Impfkristallen über Nacht im Kühlraum stehengelassen wobei das Produkt auskristallisiert. Man rührt den Kristallbrei noch einmal gut durch und saugt dann ab und wäscht zweimal mit ca. 80 ml eiskaltem Methanol nach und trocknet das farblose, kristalline Produkt im Exsiccator (N₁).
Aus der Mutterlauge läßt sich weiteres Produkt gewinnen, indem man diese auf ca. 300 ml einengt und mit ca. 600 ml Isopropanol und einigen Impfkristallen versetzt. Nach Stehenlassen über Nacht im Kühlraum erhält man weiteres kristallines Produkt, das abgesaugt und mit wenig eiskaltem Methanol nachgewaschen wird (N₂).
Ausbeute: N₁:104.9 g; N₂: 24.3 g;
Gesamt-Ausbeute: 129.2 g
R_{F}(n-Butanol/Essigsäure/Wasser/Ethylacetat = 1:1:1:1): 0.2
MS(DCl): 119 [M+H]⁺
NMR(D₂O): 2.92-3.18 ppm(m, 4H, CH₂-CH₂); 3.35 ppm(s, 2H, CH₂-CO)

### Beispiel 5:

1.80 l Diaminoethan werden in 2.61 l Methanol gelöst, und anschließend unter Kühlung und Rühren 840 g Glyoxylsäure Monohydrat gelöst in 2.61 l Wasser langsam dazugegeben. Die Mischung wird in einen Autoklaven gefüllt und mit 78 g Katalysator (Pd-Tierkohle) versetzt und bei 0.3 bar Wasserstoffdruck und Raumtemperatur hydriert. Nach Aufnahme von 200 l Wasserstoff ist die Reaktion beendet. Zur Aufarbeitung wird vom Katalysator abgesaugt und das Filtrat im Vakuum zur Trockene eingeengt. Der Rückstand wird zweimal mit Toluol (ca. 1 l) nachdestilliert und das resultierende gelbe Öl in 1.56 l Methanol aufgenommen, 3 h unter Kühlung gerührt und dann abgesaugt. Man wäscht mit wenig eiskaltem Methanol nach und trocknet das farblose, kristalline Produkt im Exsiccator (N₁). Aus der Mutterlauge läßt sich weiteres Produkt gewinnen indem man diese auf ca. 1.5 l einengt und mit ca. 3 l Isopropanol und einigen Impfkristallen versetzt. Nach Stehenlassen über Nacht im Kühlraum bei 4°C erhält man weiteres kristallines Produkt, das abgesaugt, mit wenig eiskaltem Methanol nachgewaschen und im Exsiccator getrocknet wird(N₂).
- Ausbeute:: N₁:408 g; Schmp.: 149-153°C, Zers.;
N₂: 180 g; Schmp.: 152-154°C, Zers.
Gesamt-Ausbeute: 588 g
R_{F}(n-Butanol/Essigsäure/Wasser/Ethylacetat = 1:1:1:1): 0.2
MS(DCI): 119 [M+H]⁺
NMR(D₂O): 2.92-3.18 ppm(m, 4H, CH₂-CH₂); 3.35 ppm(s, 2H, CH₂-CO)

### Beispiel 6:

### Aminoethylglycinmethylester-Dihydrochlorid: (C₅H₁₂N₂O₂ · 2HCl 205.09)

460 g Aminoethylglycin werden in 14 l Methanol eingetragen und dann 500 g HCI-Gas in die Suspension eingeleitet. Die Mischung erwärmt sich und wird anschließend insgesamt 8 h unter Rückfluß gehalten. Danach ist die Umsetzung beendet und die Mischung wird auf 0°C abgekühlt und 3 h bei dieser Temperatur nachgerührt. Man saugt über eine Nutsche ab und trocknet das Produkt im Vakuum . Man erhält 716 g farblose Kristalle vom Schmelzpunkt 190-192°C.
R_{F}(n-Butanol/Essigsäure/Wasser/Ethylacetat = 1:1:1:1): 0.3
MS(DCI): 133 [M+H]⁺
NMR(D₂O): 3.42-3.65 ppm(m, 4H, CH₂-CH₂); 3.92 ppm(s, 3H, CO-OCH₃), 4.18 ppm(s, 2H, CH₂-CO)

## Patentansprüche

1. Verfahren zur Herstellung von Aminoethylglycin, dadurch gekennzeichnet, daß man Diaminoethan mit Glyoxylsäure unter Kühlung und Rühren im Verhältnis von 2-10 : 1 in Wasser, C₁-C₃-Alkohol oder Wasser/Alkohol-Gemischen vermischt und mit Wasserstoff in Gegenwart eines Katalysators als Reduktionsmittel bei Normaldruck oder einem Druck bis 0,5 bar hydriert, anschließend das Lösungsmittel und das überschüssige Diaminoethan abdestilliert, den Rückstand mit Toluol ko-evaporiert und mit C₁-C₃-Alkyl-Alkohol behandelt, wobei Aminoethylglycin auskristallisiert.

2. Verfahren gemäß Anspruch 1, dadurch gekennzeichnet, daß als Katalysator Palladium auf Kohle eingesetzt wird.

3. Verfahren gemäß Anspruch 1 und/oder 2, dadurch gekennzeichnet, daß Diaminoethan und Glyoxylsäure im Verhältnis 3-5 : 1 eingesetzt werden.

4. Verfahren gemäß den Ansprüchen 1 bis 3, dadurch gekennzeichnet, daß bei 0,3 bar hydriert wird.

5. Verfahren gemäß den Ansprüchen 1 bis 4, dadurch gekennzeichnet, daß das entstandene Rohprodukt durch Behandeln mit Methanol kristallisiert.

## Claims

1. A process for preparing aminoethylglycine, wherein diaminoethane is mixed with glyoxylic acid, while cooling and stirring, in a ratio of 2-10 : 1, in water, C₁-C₃-alcohol or water/alcohol mixtures, and this mixture is hydrogenated with hydrogen in the presence of a catalyst as reducing agent, under standard pressure or a pressure up to 0.5 bar, the solvent and the excess diaminoethane are then distilled off, and the residue is coevaporated together with toluene and treated with C₁-C₃-alkylalcohol, whereupon aminoethylglycine crystallizes out.

2. The process as claimed in claim 1, wherein palladium on charcoal is employed as the catalyst.

3. The process as claimed in claim 1 and/or 2, wherein diaminoethane and glyoxylic acid are employed in a ratio of 3-5 : 1.

4. The process as claimed in claims 1 to 3, wherein hydrogenation is carried out at 0.3 bar.

5. The process as claimed in claims 1 to 4, wherein the resulting crude product is crystallized by being treated with methanol.

## Revendications

1. Procédé pour la préparation de l'aminoéthylglycine, caractérisé en ce que l'on mélange du diaminoéthane avec de l'acide glyoxylique, en refroidissant et sous agitation, en un rapport allant de 2:1 à 10:1, dans de l'eau, un alcool en C₁-C₃ ou des mélanges d'eau et d'alcool, et on soumet ce mélange à une hydrogénation avec, en tant que réducteur, de l'hydrogène en présence d'un catalyseur,. sous la pression normale ou sous une pression allant jusqu'à 0,5 bar, puis on élimine par distillation le solvant et le diaminoéthane en excès, on évapore le résidu avec du toluène et on traite le résidu par un alcanol en C₁-C₃, ce par quoi l'aminoéthylglycine se sépare en cristaux.

2. Procédé selon la revendication 1, caractérisé en ce que l'on utilise comme catalyseur du charbon palladié.

3. Procédé selon la revendication 1 et/ou la revendication 2, caractérisé en ce que l'on utilise le diaminoéthane et l'acide glyoxylique en un rapport allant de 3:1 à 5:1.

4. Procédé selon les revendications 1 à 3, caractérisé en ce que l'on effectue l'hydrogénation sous 0,3 bar.

5. Procédé selon les revendications 1 à 4, caractérisé en ce que le produit brut obtenu est cristallisé par traitement par du méthanol.
